# EUROPEAN PATENT SPECIFICATION

(11) **EP 1 509 370 B1**
(45) Date of publication and mention of the grant of the patent: **23.11.2005**
(21) Application number: 03727762.1
(22) Date of filing: 26.05.2003
(51) Int. Cl.: B26B 21/58, B26B 21/60

(54) **DIAMOND CUTTING INSERT**
SCHNEIDEINSATZ AUS DIAMANT
ELEMENT DE COUPE RAPPORTE A REVETEMENT DIAMANT

(30) Priority: 30.05.2002 GB 0212530
(43) Date of publication of application: 02.03.2005
(73) Proprietor: Element Six Limited, Isle of Man IM99 6AQ (GB)
(72) Inventor: Wort, Christopher John Howard, Oxfordshire OX12 7DG (GB); HALL, Clive, Edward, 6511 PC Nijmegen (NL); GODFRIED, Herman, Philip, 1081 BW Amsterdam (NL)
(74) Representative: Benson, John Everett
(86) International application number: PCT/IB2003/002005
(87) International publication number: WO 2003/101683

(56) References cited:
- WO-A-93/00204
- WO-A-98/04382
- WO-A-02/100610
- FR-A- 2 536 691
- US-B1- 6 389 699

## Description

This invention relates to a diamond cutting insert and more particularly to a diamond razor blade.

Conventional razor blades are made from steel and go blunt during the hair shaving process. Techniques to improve the longevity of steel blades include the application of hard coatings and the treatment of the steel by, for example, ion implantation. Whilst these enhancement techniques do work, the improvement in longevity (the length of time the blade remains sharp) is only modest.

It has been suggested that the longevity of a steel razor blade can be improved by coating the cutting edge of the blade with a hard material such as a ceramic.

It has also been recognised that harder materials than steel, such as ceramics, generally make longer lasting blades. The harder the material, the longer the blade will last, provided the edge does not damage or chip.

FR2 536 691 describes a diamond razor blade made from multiple single crystals of diamond each of which has a sharpened edge.

US 4,720,918 describes a steel blade having a particular profile leading up to the cutting edge thereof. The steel blade may be coated. It is also suggested in the specification that the steel blade may be replaced by harder blade material such as sapphire, titanium carbide or diamond.

### SUMMARY OF THE INVENTION

According to the present invention, a cutting insert, particularly a razor blade or surgical blade, comprises a layer of diamond having a cutting edge defined at the intersection of two converging surfaces, the converging surfaces, being coated with a layer, generally a thin layer, of diamond-like carbon (DLC).

The layer of DLC may also cover the cutting edge or may terminate just short of the cutting edge. Thus, if an extremely sharp cutting edge is desired, then the cutting edge may be provided by the diamond layer and the DLC coating terminate on each converging surface just short- of the cutting edge. Alternatively, the cutting edge may be covered by a layer of DLC. The thickness of this layer can be tailored to achieve specific objectives. If a very sharp cutting edge is desired, then the layer can be made extremely thin. Alternatively, if a less sharp cutting edge is desired, then a thicker layer of DLC can be provided thereon.

The diamond layer may be monolithic in which event the cutting edge will be continuous and uninterrupted by bonding regions. The diamond layer may also comprise a plurality of diamond pieces or segments bonded together. In this event, the cutting edge will have one or more discontinuities defined by the bonding regions. When the diamond layer is monolithic, it is preferably CVD diamond.

Diamond-like carbon (DLC) (or alpha: C or alpha: C-H as it is sometimes termed) is an amorphous material, made up of carbon and hydrogen. The structure comprises a mixture of sp³ and sp² bonded carbon with significant C-H bonding and there is no long-range order. The term diamond-like carbon arises due to its extreme hardness, e.g. Vicker's hardness as high as 6-8000 kg/mm, although this is less than that of diamond, generally 8-10 000 kg/mm².

DLC layers are generally grown under conditions of ion bombardment and are highly stressed.

The thickness of the DLC layers will vary according to the nature of the cutting insert, but will typically be less than 1 µm, more typically less than 0.5 µm. An example of a thickness range is 0.1 to 0.4 µm.

The DLC coating on the converging surfaces leading to a diamond cutting edge provides the following advantages and benefits:
1) modification of frictional properties, making it more suitable for shaving, for example;
2) controlled modification of tip radius to control sharpness and strengthen and thicken the cutting tip;
3) DLC is amorphous, so does not exhibit chipouts, and the coating suppresses chipouts in the underlying diamond edges;
4) DLC coatings have a tendency to self planarise. Thus, the final roughness of a coated surface can be less than the initial surface. This is useful in coating diamond in 'infilling' scratches and other polishing flaws.

A DLC coating to diamond also gives rise to the following advantages:
a) exceptional adhesion because of the chemical similarity between diamond and DLC;
b) diamond is well capable of supporting the intrinsic stresses of DLC. These stresses are typically tensile, putting the diamond under compressive stress and controlling brittle fracture, even in single crystal diamond;
c) the thermal expansivity and many other properties of diamond and DLC are much better matched than with conventional substrates for DLC.

### BRIEF DESCRIPTION OF THE DRAWINGS

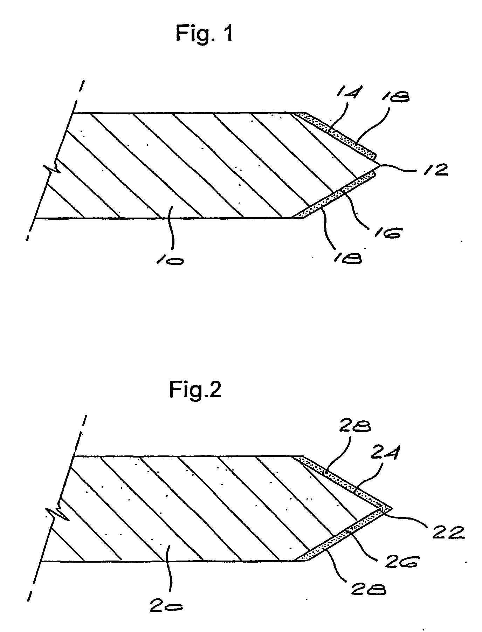

Figures 1 and 2 illustrate schematic sectional side views of two embodiments of cutting inserts of the invention.

### DETAILED DESCRIPTION OF THE INVENTION

The cutting insert of the invention may take a variety of forms and be useful in a variety of applications. Examples are:
a) A cutting insert or blade used in laser assisted surgery (LAS), comprising a diamond insert and more typically a CVD diamond insert, wherein the surfaces leading to the cutting edge and the cutting edge are coated with a layer of DLC. CVD diamond is diamond produced by chemical vapour deposition. In laser assisted surgery, the laser energy is projected through the blade to cauterise the cut tissue. In this application the DLC coating is optimised for stable, long-term non-stick properties for coagulated proteins from blood plasma (fibrines, fibrinogens), tissue fluid and red blood cells. In addition the coating provides protection during post-operative cleaning procedures used to remove body materials adherent to the blade. The thickness of the coating is typically 0.1 - 0.15 µm, providing the bulk properties of the layer desired but avoiding significant interference colours or optical absorption in the layer. In this type of application, laser induced coagulation results in the issue of material sticking to the blade being of prime concern. Thus, for this application the DLC coating is generally tailored to the softer end of its potential range, with the objective of further improving the non-stick properties of the coating, and additionally minimizing the stress in the layer and thus maximizing its adhesion. Such layers are generally more polymeric, and contain more H₂ or SiO.
b) A cutting insert used in general surgery, comprising a diamond insert and more typically a CVD diamond insert, wherein the surfaces leading to the cutting edge and the cutting edge itself are coated with a layer of DLC. Again, layers are typically 0.1 to 0.15 µm thick, providing layers free of interference colours and with good adhesion to the diamond substrate. The non-stick properties, although important are not as dominant in the coating optimisation as for the laser assisted surgery; relatively soft DLC coatings are still generally used but these can be harder than for LAS
   In application both these types of surgical blades have shown a substantially improved incision characteristic and longer lifetime between cleaning operations necessary to maintain this performance, improving the utility of the blades in application.
c) A cutting insert used for human shaving, particularly for the face, comprising a diamond insert and more typically a CVD diamond insert, coated with a layer of DLC. The DLC coating may be tailored to achieve particular interference colours, which may or may not be acceptable depending on the market, and non-stick properties to avoid the adhesion of fatty molecules and thus minimise skin drag. The wetting properties of the DLC coating may also be tailored to improve lubrication by water/soap used in the shave, for example by increasing the silica (SiO) content of the coating. Another design issue is the sharpness of the cutting tip. The uniformity of the DLC coating can be controlled so that the coating or layer is thinner or absent at the very cutting tip or edge. A similar profile can also be generated by DLC coating the blade prior to the application of a final polishing process to the cutting edge. Diamond can take a sharper edge than may be desirable for shaving. The DLC layer can be tailored to increase the cutting edge radius in a controlled manner, providing the ideal edge sharpness in addition to an edge more resistant to chipouts.

Embodiments of cutting inserts of the invention will now be described with reference to Figures 1 and 2 of the accompanying drawings. Referring to Figure 1, a cutting insert comprises a diamond layer 10 having a cutting edge 12 defined at the intersection of two converging surfaces 14, 16. Each of the converging surfaces 14, 16 is provided with a layer 18 of DLC. The layers 18 terminate just short of the cutting edge 12 which is thus a diamond cutting edge.

In the embodiment of Figure 2, a layer 20 of diamond is provided having a cutting edge 22 defined at the intersection of converging surfaces 24, 26. The converging surfaces 24, 26 and cutting edge 22 are all coated with a layer 28 of DLC.

The DLC coating may be applied to the diamond cutting edge by methods known in the art. A wide range of parameters and methods appropriate to the deposition of DLC coatings are known. Often the limitation on growth conditions for conventional DLC applications is placed by the sensitivity of the substrate, whereas diamond is tolerant to a much wider range of deposition conditions, coating thicknesses, and coating properties.

The invention is further illustrated by the following examples:

### Example 1

A diamond blade is taken and mounted into a groove in a prepared metal holder, 3 mm thick, which holds the blade at an angle of 70°C to the normal of the blade, exposing one side of the cutting edge of the blade uppermost. This metal holder is then placed onto the counter-electrode of an RF bias physical vapour deposition system, with the metal base in intimate electrical and thermal contact with this counter electrode. The deposition process can be operated over a range of conditions, which can be used to tailor the exact properties and composition of the DLC coating produced. In general the process comprises the mixing of H₂, CH₄, and possibly an inert gas such as Ar, and maintaining this mixture at a pressure in the range 1-500 mTorr (0.12 - 65 Pa) and more typically 5-50 mTorr (0.66 - 6.6 Pa). A plasma is generated using RF frequency, typically 13.56 MHz, and an electrode bias generated by use of a blocking capacitor which is related to the relative areas of the two electrodes. Typical bias voltages used lie in the range 100 - 1000 V, and more typically in the range 400 - 700 V. The substrate temperature is generally maintained at less than 200°C and preferably less than 150°C. The blade is then remounted to expose the second face of the cutting edge and the deposition process repeated.

An alternative method is the use of an ion gun, where a beam of carbon and hydrogen ions is generated and directed onto the blade in the region of the cutting edge.

### Example 2

The outline dimensions of a number of blades were cut with a high power laser, out of a plate of polycrystalline CVD diamond. The blades were polished to the appropriate thickness (0.6 mm). The technique of laser cutting diamond is well known in the field. Subsequently two facets were polished onto each blade, the intersection of the facets forms a cutting edge of the blade. Polishing was done by pressing the blade at an angle onto a scaife made from steel or cast iron or epoxy resin with diamond particles embedded in the resin as is well known in the art. Other ways to produce the blades are to laser cut the facets in the plates and subsequently polish these laser-cut facets. At this point the blades were shaped ready for coating.

In order to obtain a strongly adherent coating the surfaces of the blades were cleaned, to remove all dirt and and adsorbed matter (both organic and inorganic in nature). Cleaning was generally performed using a concentrated acid such as nitric, sulfuric, or hydrochloric acid, followed by a rinse in demineralised water, a further rinse in an alcohol such as methanol, ethanol, or propanol, and oven drying at a temperature of 60-100°C. In one particular example concentrated sulphuric acid was used followed by rinsing in water and propanol and the blades were dried at 60°C for 1 hour.

As a further cleaning stage, the blades were exposed to a microwave plasma discharge which contained a high concentration of oxygen radicals. These oxygen radicals react with any remaining contaminants on the surface of the diamond and remove them. This etch can also attack the diamond itself, so the blade was only exposed to the plasma for a short period of time, typically less than 1/2 hour. In one case the blade was exposed to a microwave (2.45 GHz) generated plasma for 15 minutes only and the oxygen gas pressure in the reaction chamber was 1 mbar (1 x 10² Pa). The discharge power was approximately 600 Watt peak power with a duty cycle of approximately 20%.

Following the etch, a DLC coating was deposited on the facets and cutting edges of the blades. This was done using a mixture of gases comprising a carbon source, hydrogen, and an oxygen source to a commercially available recipe. In some cases the mixture also contained small amounts of a silicon source such as silane to introduce some silica into the DLC coating. The gases where then broken down by the microwave plasma and a layer of DLC typically about 200 nm thickness was applied to the facets and cutting edge of the blade. In other cases, thicker DLC coatings were applied up to approximately 4 microns thickness although in general there is limited benefit from DLC coatings greater than 1 µm thickness. The Vickers hardness of the DLC coating was approximately 1200-1500 VH.

As fatty materials (such as lipids and other fatty materials in the human skin) have very weak adhesion to DLC-type layers, while on the other hand they strongly adhere to diamond, the DLC coated blades give significantly less drag of the skin during shaving than uncoated diamond blades. Friction tests that were done for common materials such as metals indicate that the DLC coated diamond has a friction coefficient significantly lower than uncoated diamond. In one example a friction coefficient for the DLC coated diamond on steel was measured to be less than 0.05, while the uncoated diamond had friction coefficients in the range of 0.4 to 0.8.

Since the DLC coating has a diamond-like structure the adherence to diamond is exceptionally strong and this combined with inherent strength of diamond leads to exceptional long lifetime of the coating. The addition of silica in some of the DLC layers was intended to reduce the stress within the layers and improve adhesion and wear characteristics even further. In tests it was not possible to remove the DLC coating from the diamond surface by mechanical means other than by (abrasive) polishing techniques such as those used for preparing the blades. Coating roughness was very low (typically this is less than 0.2 nm) thus adding little or nothing to the original roughness of the polished blade which was 2 nm or less, and in some instances the overall roughness was reduced.

Control of the frictional properties of the blade with the skin does not require the coating to extend to the cutting edge. Such blades were fabricated. The simplest method of fabrication is to prepare mechanically the final cutting edge after DLC coating by removing the DLC at the cutting edge. A particular advantage of this configuration is that diamond wear at the precise cutting tip can be lower than that of the DLC coating.

However, other benefits such as suppression of chipouts are obtainable or enhanced by coating very close to or around the diamond cutting edge. The effect on the cutting geometry is dependent on the relative dimensions of the cutting tip radius and the coating thickness at this point, although it is also possible to shape the DLC coated cutting edge subsequent to applying the coating.

## Claims

1. A cutting insert comprising a layer of diamond having a cutting edge defined at the intersection of two converging surfaces, the converging surfaces being coated with a layer of diamond-like carbon (DLC).

2. A cutting insert according to claim 1 wherein the DLC layer also coats the cutting edge.

3. A cutting insert according to claim 1 or claim 2 which is a razor blade.

4. A cutting insert according to claim 1 or claim 2 which is a surgical blade.

5. A cutting insert according to any one of the preceding claims wherein the layer of DLC is a thin layer.

6. A cutting insert according to any one of the preceding claims wherein the thickness of the DLC layer is less than 1 µm.

7. A cutting insert according to any one of the preceding claims wherein the thickness of the DLC layer is less than 0.5 µm.

8. A cutting insert according to any one of the preceding claims wherein the thickness of the DLC layer is in the range 0.1 to 0.4 µm.

9. A cutting insert according to any one of the preceding claims wherein the diamond layer is monolithic.

10. A cutting insert according to any one of claims 1 to 8 wherein the diamond layer comprises a plurality of pieces or segments bonded together.

11. A cutting insert according to any one of the preceding claims wherein the DLC coating, contains SiO.

## Patentansprüche

1. Schneideinsatz, der eine Diamantschicht mit einer Schneidkante an dem Schnittpunkt von zwei zusammenlaufenden Oberflächen aufweist, wobei die zusammenlaufenden Oberflächen mit einer Schicht aus *diamond-like carbon* (DLC) überzogen sind.

2. Schneideinsatz nach Anspruch 1, **dadurch gekennzeichnet, dass** die DLC-Schicht auch die Schneidkante überzieht.

3. Schneideinsatz nach einem der Ansprüche 1 oder 2, **dadurch gekennzeichnet, dass** es eine Rasierklinge ist.

4. Schneideinsatz nach einem der Ansprüche 1 oder 2, **dadurch gekennzeichnet, dass** es eine chirurgische Klinge ist.

5. Schneideinsatz nach einem der vorstehenden Ansprüche, **dadurch gekennzeichnet, dass** die DLC-Schicht eine dünne Schicht ist.

6. Schneideinsatz nach einem der vorstehenden Ansprüche, **dadurch gekennzeichnet, dass** die Dicke der DLC-Schicht weniger als 1 µm beträgt.

7. Schneideinsatz nach einem der vorstehenden Ansprüche, **dadurch gekennzeichnet, dass** die Dicke der DLC-Schicht weniger als 0,5 µm beträgt.

8. Schneideinsatz nach einem der vorstehenden Ansprüche, **dadurch gekennzeichnet, dass** die Dicke der DLC-Schicht im Bereich von 0,1 bis 0,4 µm liegt.

9. Schneideinsatz nach einem der vorstehenden Ansprüche, **dadurch gekennzeichnet, dass** die Diamantschicht monolithisch ist.

10. Schneideinsatz nach einem der Ansprüche 1 bis 8, **dadurch gekennzeichnet, dass** die Diamantschicht eine Vielzahl miteinander verbundener Stücke oder Segmente aufweist.

11. Schneideinsatz nach einem der vorstehenden Ansprüche, **dadurch gekennzeichnet, dass** die DLC-Beschichtung SiO enthält.

## Revendications

1. Plaquette de coupe comprenant une couche de diamant ayant un bord tranchant défini à l'intersection de deux surfaces convergentes, les surfaces convergentes étant recouvertes d'une couche de diamant de dépôt CDA (DLC).

2. Plaquette de coupe selon la revendication 1 dans laquelle la couche DLC couvre également le bord tranchant.

3. Plaquette de coupe selon la revendication 1 ou la revendication 2 qui est une lame de rasoir.

4. Plaquette de coupe selon la revendication 1 ou la revendication 2 qui est une lame chirurgicale.

5. Plaquette de coupe selon l'une quelconque des revendications précédentes dans laquelle la couche de DLC est une couche fine.

6. Plaquette de coupe selon l'une quelconque des revendications précédentes dans laquelle l'épaisseur de la couche DLC est inférieure à 1 µm.

7. Plaquette de coupe selon l'une quelconque des revendications précédentes dans laquelle l'épaisseur de la couche DLC est inférieure à 0,5 µm.

8. Plaquette de coupe selon l'une quelconque des revendications précédentes dans laquelle l'épaisseur de la couche DLC est dans la fourchette de 0,1 à 0,4 µm.

9. Plaquette de coupe selon l'une quelconque des revendications précédentes dans laquelle la couche de diamant est monolithique.

10. Plaquette de coupe selon l'une quelconque des revendications 1 à 8 dans laquelle la couche de diamant comprend une pluralité de pièces ou segments liés les uns aux autres.

11. Plaquette de coupe selon l'une quelconque des revendications précédentes dans laquelle la couche DLC contient du SiO.
